**Europäisches Patentamt**

**European Patent Office**

**Office européen des brevets**

(19)

(11) Veröffentlichungsnummer: **0 150 721**
**A2**

(12)

# EUROPÄISCHE PATENTANMELDUNG

(21) Anmeldenummer: 85100144.6

(22) Anmeldetag: 09.01.85

(51) Int. Cl.⁴: **A 61 K 31/55**
**// (A61K31/55, 31:495)**

---

(30) Priorität: 25.01.84 DE 3402507

(43) Veröffentlichungstag der Anmeldung: 07.08.85
Patentblatt 85/32

(84) Benannte Vertragsstaaten: **AT BE CH FR GB LI NL SE**

(71) Anmelder: **Paliz, Peter M. Forrer, Lange Gasse 34, Basel (CH)**

(72) Erfinder: **Kessler, Renate, Skablosenstrasse 2, D-8000 München 50 (DE)**

(74) Vertreter: **Dr. E. Wiegand Dipl.-Ing. W. Niemann Dr. M. Kohler Dipl.-Ing. J. Glaeser Dr. H.-R. Kressin Patentanwälte, Herzog-Wilhelm-Strasse 16, D-8000 München 2 (DE)**

---

(54) **Arzneimittel.**

(57) Die Erfindung betrifft ein Arzneimittel, das neben üblichen Hilfs- und/oder Trägerstoffen als Wirkstoff eine Kombination von Cinnarizin und wenigstens eine Benzodiazepin-Verbindung enthält.

EP 0 150 721 A2

0150721

## Beschreibung

Die Erfindung betrifft ein Arzneimittel, enthaltend eine Kombination von Cinnarizin mit wenigstens einem Benzodiazepin.

Benzodiazepine werden als Ataraktika und Anxiolytika zur Bekämpfung neurotischer Achsensymptome angewendet. Sie zeigen eine geringe Toxizität.

Benzodiazepine wirken vorwiegend durch Anhäufung von aktiven Metaboliten im Zentralnervensystem. Dies führt zu einem relativ lang anhaltenden therapeutischen Effekt bei verhältnismäßig niedrigen Dosen. Die unterschiedliche Eliminationszeit der einzelnen Benzodiazepine bestimmt deren sedative oder hypnotische Eigenschaft. Typische Vertreter mit sedativen Eigenschaften sind Chlordiazepoxid, Diazepam, Prazepam, Oxazepam und Bromazepam. Hypnotische Eigenschaften besitzen Nitrazepam, Flurazepam und Flunitrazepam. Zusätzlich antiepileptische Eigenschaften besitzen Clonazepam und Diazepam.

Neben dem gestörten Gleichgewicht zwischen den Erregungsprozessen und der Hemmung emotionaler Reaktionen, welches einerseits zu Neurosen und andererseits zu Schlafstörungen führt, existieren noch andere wesentliche Faktoren, die den Schlaf indirekt beeinflussen. Zu diesen zählen cerebrale Durchblutungsstörungen, atherosklerotische Veränderungen sowie Vasokonstriktion oder Minderdurchblutung des Cerebrums. Alle Formationen des Zentralnervensystems sind auf Sauerstoffmangel äußerst empfindlich und reagieren im Falle eines solchen mit heftigen Funktionsstörungen.

Wie nun überraschend gefunden wurde, kann die Wirkung von Benzodiazepinen durch gleichzeitge Gaben von Cinnarizin potenziert werden. Das Cinnarizin wird z.B. bei cerebralen Durchblutungsstörungen verwendet. Nach neueren Erkenntnissen besitzt die Substanz auch **calc**iumantagonistische Wirkung.

Die synergistische bzw. potenzierende Wirkung von Cinnarizin auf Benzodiazepine wurde durch Messung der Spontan-Motorik an der Maus ermittelt. Die Motorik der Tiere wird hierbei über 19 Fotozellen am Boden des Mobilitäts-Messgerätes in Form von Impulsen gezählt. Um zufallsbedingte Mobilitäts-einflüsse auszuschalten, wurden die Tests unter streng kontrollierten Bedingungen durchgeführt, die darin bestanden, dass nur 5 Tiere pro Messgerät eingesetzt wurden, dass alle Tests zur gleichen Tageszeit parallel unter optimalen und gleichbleibenden Temperaturbedingungen durchgeführt wurden, alle Tiere derselben Population entstammten, vor Versuchsbeginn 14 Tage in einem gemeinsamen Käfig gehalten wurden und innerhalb dieser Vorbereitungsphase zur Gewöhnung täglich über 150 min im Mobilitätsmessgerät verbrachten.

Aus der Gruppe der Benzodiazepine, deren Klassifizierung üblicherweise nach Substanzen mit langer, mittlerer und kurzer Halbwertszeit erfolgt, wurden als typische Gruppenvertreter für den Versuch das Diazepam (lange Halbwertszeit), das Bromazepam (mittlere Halbwertszeit) und das Oxazepam (kurze Halbwertszeit) ausgewählt. Die orale $ED_{50}$ dieser Substanzen wurde nach gleicher Methodik und identischen Kriterien getrennt bestimmt. Die graphisch aus der Dosis-Wirkungskurve ermittelte orale $ED_{50}$ betrug 3.2 mg/kg für Diazepam, 2.42 mg/kg für Bromazepam und 1.55 mg/kg für Oxazepam. Nach gleicher Methodik wurden die anderen Benzodiazepine getestet.

Die nach 1 h im Messgerät ermittelte Impulszahl wurde auf 100% gesetzt. Danach erhielten die Tiere der Kontrollgruppe p.o. das Vehikel (0.5% Methylcelluloselösung mit 2.5% Tween 20), die Tiere der Testgruppe die Testsubstanz gelöst in Vehikel. Die Ergebnisse sind in **der Tabelle zusammengestellt.**

Wie die Testergebnisse zeigen, ist die gemessene Dämpfung der Spontan-Motorik durch die Benzodiazepin-Cinnarizin Kombination im Vergleich zur alleinigen Verabfolgung gleicher Dosen des Benzodiazepin wesentlich stärker und setzt auch zeitlich deutlich früher ein.

Cinnarizin hat gemäß Eksp.Med.Morfol. <u>19</u> (1980), Seiten 212-216 nur einen schwachen tranquillisierenden Effekt. Die nach Cinnarizin-Gabe allein erhaltenen Messwerte schwanken im Bereich der Kontrollwerte und deuten eher auf einen geringen stimulierenden als dämpfenden Effekt, der aus der cerebralen Wirkung dieser Substanz erklärbar ist. Zudem konnte in getrennten Experimenten zur Verhaltensunterdrückung, dem eigentlichen Test für sedativ wirksame Verbindungen, mit Cinnarizin selbst bis zu 300 mg/kg p.o. an der Maus kein Effekt erzielt werden.

Der positive Effekt von Cinnarizin auf die getesteten Benzodiazepine ist unabhängig von deren Halbwertszeit messbar. Unterschiedlich ist offensichtlich der Einfluss des Mengenanteils von Cinnarizin, der nur im Falle der Kombination mit Oxazepam eine länger anhaltende Wirkung bei höherer Cinnarizindosis zeigt. Bei den Benzodiazepinen mit mittlerer (Bromazepam) und längerer (Diazepam) Halbwertszeit bewirkt die höhere **Cinnarizindosis gegen Ver**suchsende eine deutliche Motilitätssteigerung gegenüber den Werten bei alleiniger Benzodiazepin-Dosierung.

0150721

Aus den Ergebnissen der **Tabelle geht ferner hervor,** daß **zur**
Erzielung gleicher therapeutischer Wirkung durch Anwendung der
Kombination eines Benzodiazepins mit Cinnarizin die Dosis des
Benzodiazepins verringert werden kann.

Die erfindungsgemässen Arzneimittel werden rektal in Form
von Rektalkapseln oder Supporitorien und oral in Form von
Tabletten, Dragees, Hart- oder Weichgelatinekapseln unter
Verwendung üblicher pharmazeutischer Hilfsmittel, sowie
gebräuchlicher Träger, Spreng- und Gleitmittel verabreicht.

Die erfindungsgemässen Arzneimittel enthalten ein Benzodiazepin (I) und Cinnarizin (II) in einem Gewichtsverhältnis
von I:II von 1:3 bis 1:50, vorzugsweise 1:5 bis 1:10. Zweckmässigerweise enthält das erfindungsgemässe Arzneimittel pro
Dosiseinheit 2-25 mg der Benzodiazepinkomponente (I) und
10-75 mg Cinnarizin.

Tabelle Spontan-Motorik nach oraler Applikation an der Maus; mittlerer Prozentwert bezogen auf den jeweiligen Gruppen-Nullwert von 10 Tieren (2 Tests je 5 Tiere/Gruppe)

| | | mg/kg | 0 | 15 | 30 | 45 | 60 | 90 | 120 | 150 min p.a. |
|---|---|---|---|---|---|---|---|---|---|---|
| Exp. 1 | Kontrolle | - | 100 | 67.7 | 46.8 | 56.7 | 40.3 | 31.1 | 39.8 | - |
| | Cinnarizin | 20 | 100 | 70.5 | 50.1 | 52.3 | 44.6 | 46.7 | 52.4 | - |
| | | 100 | 100 | 68.4 | 49.6 | 51.1 | 48.2 | 42.3 | 49.3 | - |
| | Oxazepam | 1.55 | 100 | 53.3 | 78.9 | 21.4 | 14.1 | 30.7 | 12.4 | - |
| Exp. 2 | Kontrolle | - | 100 | 54.5 | 41.6 | 50.8 | 34.5 | 50.2 | 56.8 | - |
| | Oxazepam + Cinnarizin | 1.55 + 20.0 | 100 | 60.3 | 15.3 | 10.6 | 13.0 | 13.7 | 19.4 | - |
| Exp. 3 | Kontrolle | - | 100 | 59.5 | 51.2 | 43.8 | 38.2 | 68.0 | 68.5 | - |
| | Oxazepam + Cinnarizin | 1.55 + 100.0 | 100 | 38.8 | 12.3 | 13.6 | 8.4 | 32.3 | 9.5 | - |
| Exp. 4 | Bromazepam | 2.42 | 100 | 46.5 | 45.7 | 33.0 | 29.5 | 16.4 | 16.2 | 23.5 |
| | Bromazepam + Cinnarizin | 2.42 + 20.0 | 100 | 18.2 | 6.8 | 6.2 | 8.4 | 12.6 | 17.8 | 25.2 |
| | Bromazepam + Cinnarizin | 2.44 + 100.0 | 100 | 20.6 | 7.2 | 6.1 | 10.8 | 14.5 | 20.6 | 44.5 |

Tabelle (Forts.)

| | | mg/kg | 0 | 15 | 30 | 45 | 60 | 90 | 120 | 150 min. |
|---|---|---|---|---|---|---|---|---|---|---|
| Exp. 4 | Kontrolle | – | 100 | 63.6 | 36.4 | 34.6 | 34.1 | 69.5 | 66.3 | 55.1 |
| (Forts.) | Diazepam | 3.2 | 100 | 39.4 | 41.7 | 29.9 | 26.6 | 10.5 | 15.6 | 19.8 |
| | Diazepam + Cinnarizin | 3.2 + 20.0 | 100 | 13.3 | 3.4 | 5.7 | 7.3 | 15.1 | 19.4 | 21.7 |
| | Diazepam + Cinnarizin | 3.2 + 150.0 | 100 | 18.1 | 2.5 | 11.6 | 9.8 | 19.0 | 22.7 | 40.0 |
| Exp.5 | Chlordiazepoxid | 3.4 | 100 | 42.5 | 43.3 | 34.8 | 33.5 | 14.4 | 16.6 | 20.4 |
| | Chlordiazepoxid + Cinnarizin | 3.4 + 20.0 | 100 | 15.6 | 6.4 | 5.8 | 6.6 | 16.8 | 17.2 | 23.2 |
| Exp.6 | Prazepam | 3.05 | 100 | 44.0 | 36.5 | 32.2 | 28.6 | 13.2 | 12.5 | 18.6 |
| | Prazepam + Cinnarizin | 3.05 + 20.0 | 100 | 16.4 | 5.5 | 4.9 | 5.2 | 13.8 | 18.4 | 20.5 |
| Exp. 7 | Flurazepam | 2.65 | 100 | 47.4 | 40.8 | 35.2 | 26.5 | 21.2 | 22.4 | 24.6 |
| | Flurazepam + Cinnarizin | 2.65 + 20.0 | 100 | 20.6 | 8.4 | 6.8 | 7.8 | 9.6 | 12.8 | 20.5 |

Tabelle (Forts.)

| | | mg/kg | 0 | 15 | 30 | 45 | 60 | 90 | 120 | 150 min |
|---|---|---|---|---|---|---|---|---|---|---|
| Exp.8 | Clonazepam | 2.15 | 100 | 40.4 | 36.6 | 33.8 | 24.4 | 19.2 | 19.6 | 22.5 |
| | Clonazepam + Cinnarizin | 2.15 + 20.0 | 100 | 19.5 | 14.3 | 9.6 | 6.4 | 8.5 | 10.8 | 24.2 |
| Exp.9 | Flunitrazepam | 1.94 | 100 | 42.5 | 34.2 | 29.8 | 26.5 | 22.4 | 22.6 | 26.5 |
| | Flunitrazepam + Cinnarizin | 1.94 + 20.0 | 100 | 16.4 | 9.5 | 6.6 | 7.2 | 10.8 | 16.5 | 23.2 |
| Exp.lo | Nitrazepam | 1.80 | 100 | 44.6 | 42.4 | 39.6 | 24.5 | 19.6 | 18.4 | 19.8 |
| | Nitrazepam + Cinnarizin | 1.80 + 20.0 | 100 | 45.4 | 26.2 | 21.5 | 13.6 | 9.8 | 10.2 | 28.5 |

Beispiele

1. Hartgelatinekapsel

| | |
|---|---|
| Diazepam | 4,0 mg |
| Cinnarizin | 20,0 mg |
| Laktose D20 | 18,0 mg |
| Magnesiumstearat | 0,8 mg |
| Talkum | 3,0 mg |
| Polyvinylpyrrolidon | 2,5 mg |
| Maisstärke | 7,2 mg |

2. Tabletten

| | |
|---|---|
| Bromazepam | 5,0 mg |
| Cinnarizin | 50,0 mg |
| Hydroxypropylcellulose | 5,0 mg |
| mikrokristalline Cellulose | 33,0 mg |
| Gelatine | 2,8 mg |
| Stearinsäure | 0,3 mg |
| Talkum | 2,0 mg |

3. Suppositorien

| | |
|---|---|
| Chlordiazepoxid | 12,0 mg |
| Cinnarizin | 72,0 mg |
| Suppositorienmasse H15 | 320,0 mg |
| Suppositorienmasse W35 | 640,0 mg |

Patentansprüche

1. Arzneimittel,
   d a d u r c h   g e k e n n z e i c h n e t ,
   daß es neben üblichen pharmazeutischen Hilfs- und Trägerstoffen als Wirkstoff eine Kombination von
   a) wenigstens einer Verbindung aus der Gruppe der
      Benzodiazepine sowie
   b) Cinnarizin
   enthält.

2. Arzneimittel nach Anspruch 1, dadurch gekennzeichnet,
   daß es als Benzodiazepin Chlordiazepoxid, Diazepam,
   Prazepam, Bromazepam, Clonazepam, Oxazepam, Nitrazepam,
   Flurazepam und/oder Flunitrazepam enthält.

3. Arzneimittel nach Anspruch 1 oder 2, dadurch gekennzeichnet, daß es das Benzodiazepin und Cinnarizin in
   einem Gewichtsverhältnis von 1:3 bis 1:5o, vorzugsweise 1:5 bis 1:10, enthält.

4. Arzneimittel nach einem der vorhergehenden Ansprüche,
   dadurch gekennzeichnet, daß es für die orale oder
   rektale Applikation formuliert ist.

5. Arzneimittel nach einem der vorhergehenden Ansprüche,
   dadurch gekennzeichnet, daß es je Dosiseinheit 2-25 mg
   der Benzodiazepin-Komponente enthält.